(19) Europäisches Patentamt
European Patent Office
Office européen des brevets

(11) **EP 3 129 006 B1**

(12) # EUROPEAN PATENT SPECIFICATION

(45) Date of publication and mention of the grant of the patent:
**27.01.2021 Bulletin 2021/04**

(21) Application number: **15735733.6**

(22) Date of filing: **08.04.2015**

(51) Int Cl.:
***A61K 9/16*** *(2006.01)* ***A61K 9/20*** *(2006.01)*
***A61K 9/28*** *(2006.01)* ***A61K 31/397*** *(2006.01)*

(86) International application number:
**PCT/IB2015/052552**

(87) International publication number:
**WO 2015/155711 (15.10.2015 Gazette 2015/41)**

(54) **IMMUNOSUPPRESSANT FORMULATION**
IMMUNSUPPRESSIVE FORMULIERUNG
FORMULATION D'IMMUNOSUPPRESSEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.04.2014 US 201461977806 P**

(43) Date of publication of application:
**15.02.2017 Bulletin 2017/07**

(73) Proprietor: **Novartis AG**
**4056 Basel (CH)**

(72) Inventors:
- **BOUILLOT, Philippe Michel Rene**
**4002 Basel (CH)**
- **REYNAUD, Emeric**
**4002 Basel (CH)**

(74) Representative: **Wiessner, Michael**
**Novartis Pharma AG**
**Patent Department**
**Postfach**
**4002 Basel (CH)**

(56) References cited:
**WO-A1-2012/093161** **WO-A1-2012/095853**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 129 006 B1

## Description

### Field of the Invention

The present invention relates to a dosage form of siponimod (BAF312) and pharmaceutically acceptable salts thereof, wherein the dosage form is preferably obtainable by a specific blending process or comprises a specific amounts of compounds. Further, the present invention relates to a method for producing the dosage form and the use of agglomerates comprising siponimod and moisture protective agent for producing a storage-stable dosage form.

### Background

Siponimod (BAF312) is a sphingosine-1-phosphate (S1P) receptor modulator. SIP receptors belong to a family of closely related, lipid activated G-protein coupled receptors. S1P1, S1P3, S1P2, S1P4, and S1P5 (also respectively termed EDG-1, EDG-3, EDG-5, EDG-6 and EDG-8) are identified as receptors specific for SIP. Certain SIP receptors are associated with diseases mediated by lymphocyte interactions, for example in transplantation rejection, autoimmune diseases, inflammatory diseases, infectious diseases and cancer.

WO 2012/093161 A1 discloses a solid phase pharmaceutical composition comprising 1-{4-[1-(4-cyclohexyl-3-trifluoromethyl-benzyloxyimino)-ethyl]-2-ethyl-benzyl}-azetidine-3-carboxylic acid (BAF312, siponimod) or a pharmacologically acceptable salt, solvate or hydrate thereof, wherein the API is not exposed to a basic compound. Due to the non-exposure to basic compounds, the stability of siponimod in the composition can be increased.

However, there is still a need of further enhancing the stability of siponimod in pharmaceutical applications.

Thus, it is an object of the present invention to provide a dosage form, preferably a tablet, which stabilizes the contained siponimod, so that the active pharmacological ingredient is provided even after a prolonged storage period. Further, a dosage form having an advantageous content uniformity should be provided.

### Summary of the Invention

According to the present invention, the above objective is unexpectedly overcome by a dosage form, obtained by a process comprising the steps described in this invention. Furthermore, the above drawbacks can be overcome by a specific process for preparing a dosage form comprising siponimod by pre-blending the active pharmacological ingredient with a moisture-protective agent.

Thus, the subject of the present invention is a dosage form, obtainable by a process comprising the steps

i) providing siponimod,
ii) pre-blending the compound of step i) with moisture protective agent and, optionally, pharmaceutical excipient,
iii) blending the mixture of step ii) with pharmaceutical excipient(s),
iv) processing the mixture of step iii) to a dosage form,
v) optionally, film-coating the dosage form.

It was unexpectedly found that in the dosage form of the present invention siponimod is stabilized. At the same time, the process of dosage form formation is facilitated due to the pre-blending of siponimod with a moisture-protective agent, as the compressibility of the mixture is surprisingly increased.

Further, the invention relates to a dosage form containing 0.2 to 12% (w/w) siponimod, 1 to 8% (w/w) of moisture-protective agent and 80 to 98.8 % (w/w) of pharmaceutical excipient(s).

Further, the invention relates to a process for producing a dosage form comprising the steps

i) providing siponimod,
ii) pre-blending the compound of step i) with moisture-protective agent and, optionally, pharmaceutical excipient,
iii) blending the mixture of step ii) with pharmaceutical excipient(s),
iv) processing the mixture of step iii) to a dosage form,
v) optionally, film-coating the dosage form.

Further, the invention relates to the use of agglomerates comprising siponimod and moisture protective agent for producing a solid oral dosage form having a shelf life of at least 2 years at room temperature, i.e. 25 °C. Preferably, the agglomerates can be prepared as described below, e.g. by blending of siponimod and moisture protective agent.

## Brief Description of the Figures

**Figure 1** is a flow diagram of one embodiment of the blending process as part of the process of forming a dosage form according to the present invention.

## Detailed Description of the Invention

For the avoidance of doubt, it is hereby stated that the information disclosed earlier in this specification under the heading "Background" is relevant for the invention and is to be read as part of the disclosure of the invention.

Throughout the description and claims of this specification, the words "comprise" and "contain" and variations thereof mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification (which term encompasses both the description and the claims) is to be understood as contemplating plurality as well as singularity, unless the context otherwise requires.

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed may be combined in any combination, except combinations where at least some of such features and/or steps are mutually exclusive. The invention is not restricted to the details of any foregoing embodiments. The invention extends to any novel one, or any novel combination of the features disclosed in this specification (including any accompanying claims, abstract and drawings), or to any novel one, or any novel combination of the steps of any method or process so disclosed.

"Siponimod" as used herein is understood to comprise the compound of formula (I)

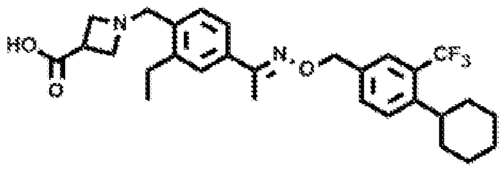

(I)

as well as the pharmaceutically acceptable salts, polymorphs, solvates and/or hydrates thereof. Siponimod as used herein has the IUPAC-name 1-{4-[1-((*E*)-4-cyclohexyl-3-trifluoromethyl-benzyloxyimino)-ethyl]-2-ethyl-benzyl}-azetidine-3-carboxylic acid (BAF312).

In a preferred embodiment of the invention, siponimod is present in the form of crystalline siponimod or siponimod salt. In a most preferred embodiment of the invention siponimod is present as crystalline siponimod hemifumarate.

The term "crystalline" can be used in the context of this invention to describe the state of a solid substance, whose constituent atoms, molecules, or ions are arranged in an ordered pattern extending in all three spatial dimensions.

The siponimod in the pharmaceutical composition form of the invention may consist of purely crystalline siponimod. Alternatively, it may also contain small amounts of non-crystalline siponimod components. In an embodiment of the invention, the siponimod contained in the inventive dosage form can be 85 to 99.999 %, more preferably 90 to 99.99 %, most preferably 95 to 99.9 % by weight crystalline siponimod.

In a preferred embodiment, the dosage form of the present invention is a solid oral dosage form, preferably a capsule or tablet, in particular, a tablet.

In a particularly preferred embodiment, the dosage form of the invention comprises siponimod as the sole pharmaceutically active agent.

In step i) according to the present invention, siponimod is provided. As described above, siponimod can be provided as a free base, as a pharmaceutically acceptable salt, polymorph, as solvate or hydrate thereof. In a preferred embodiment it is provided as siponimod hemifumarate. Further, siponimod is preferably provided in particulate form. Siponimod is preferably provided in an amount of 0.2 to 12 wt.%, preferably 1.3 to 12 wt.%, more preferably 1.3 to 3.0 wt.%, especially 2.0 to 2.7 wt.% siponimod, based on the total weight of the dosage form.

Generally, the amounts of the components of the dosage form of the present invention given in wt.% are preferably referred to the dosage form without film coating.

Further, siponimod is preferably provided in an amount of 0.1 to 10 mg preferably 0.2 to 5 mg, in particular, 1

or 2 mg, based on the amount of siponimod in form of the free base. In particular, the dosage form of the present invention comprises 2 mg siponimod. Since the active agent can be present in the form of a salt, the amount of the respective acid has to be added accordingly.

The siponimod comprised in the dosage form of the present invention can preferably have an average particle size X90 of 5 to 100 μm, more preferably 15 to 80 μm, even more preferably 30 to 70 μm, most preferably 40 to 50 μm.

The particle size X90, which is also denoted as X90 value of the integral volume distribution, is defined in the context of this invention as the particle diameter at which 90 per cent by volume of the particles have a smaller diameter than the diameter which corresponds to the X90 value. Likewise, 10 per cent by volume of the particles have a larger diameter than the X90 value. The X10 and X50 values are defined accordingly.

Further, the siponimod comprised in the dosage form of the present invention can preferably have an average particle size X50 of 1 to 25 μm, more preferably 2 to 22 μm, even more preferably 4 to 20 μm, especially preferred 5 to 17 μm.

Yet further, the siponimod comprised in the dosage form of the present invention can preferably have a particle size X10 of 0.5 to 5 μm, more preferably 1 to 4 μm, even more preferably 1.2 to 3 μm, especially preferably 1.5 to 2.7 μm.

In a preferred embodiment the ratio X90/X50 can be 1.0 to 100, preferably 1.2 to 10, more preferably 2.0 to 5.0, most preferably 2.5 to 4.0 In a preferred embodiment the ratio X50/X10 can be 1.1 to 10, preferably 1.2 to 5, more preferably 2.5 to 4.0.

The particle size distribution by volume can be measured by using laser diffractrometry. In particular, it can be measured by using the Sympatec Helos device (from Sympatec GmbH, Germany), using the Cuvette dispersion device. To carry out the measurement, a stock dispersion was prepared by mixing the drug substance with a dispersing aid (Octastat® 5000 (Octel corp)), using a vortex until a smooth and homogeneous paste was formed. The paste was then diluted and mixed to a final volume of 3 to 6 ml using white spirit. The optical concentration of the final solution was kept below 5%. The percentage values were calculated from the mean cumulative volume size curve by the software of the Sympatec instrument.

The particulate siponimod can be obtained by milling, such as wet-jet milling, pin-milling, wet-ball milling. Where the siponimod particles are derived from coarse siponimod particle crystals, the coarse crystals may be obtained e.g. by methodologies set out in WO2010/071794, WO2010/08045 or WO2010/080409.

In step ii) of the present invention, siponimod is pre-blended with moisture protective agent and, optionally, pharmaceutical excipient, e.g. a filler. The feature "pre-blending" can refer to mixing solid and/or particulate siponimod with solid and/or particulate moisture protective agent, e.g. in devices as described below. Preferably, the pre-blending leads to agglomerates comprising siponimod and moisture protective agents. It has been unexpectedly found that these agglomerates provide advantageous storage properties, i.e. they can be used for providing storage-stable dosage forms of siponimod. In one embodiment, the pre-blending with a moisture protective agent does not lead to a controlled release-type formulation of siponimod.

Generally, the moisture protective agent is a pharmaceutical excipient, capable of adhering to the siponimod particles and of protecting them from moisture during the subsequent process steps. "Adhering" as used herein has to be understood in a broad sense. The term may encompass the adsorption or agglomeration of particles. It also may encompass the agglomeration of particles of different size, i.e. larger particles of moisture protective agent and/or filler agglomerate with smaller particles of siponimod.

In one embodiment of the invention, the moisture protective agent is a hydrophobic compound or a compound comprising a hydrophobic residue. In a preferred embodiment, the moisture protective agent is a compound, preferably a pharmaceutical excipient, having a n-octanol/water partition coefficient (*log P*) of 0.1 to 20, preferably 1 to 15, more preferably of 5 to 13, in particular, 6 to 12, especially 7 to 11.

The preferred method of *log P* determination is the *shake-flask method,* which consists of adding 1 g of moisture protective agent to a volume of 10 ml n-octanol and 10 ml distilled water, respectively, at 25 °C. Subsequently, the dissolved concentration of the solute in each solvent is measured by UV/VIS spectroscopy.

The partition coefficient is the ratio of concentrations of un-ionized solute (moisture protective agent) between the two solutions. The logarithm of the ratio of the concentrations in the solvents is called log P.

- $$\log\ P_{\text{oct/wat}} = \log\left(\frac{[\text{solute}]_{\text{octanol}}}{[\text{solute}]_{\text{water}}^{\text{un-ionized}}}\right)$$

Further, the moisture protective agent can be a waxy compound. Waxy compounds can be organic materials, e.g. with a molecular weight of 100 to 2000 g/mol. Waxy compounds are preferably solid at room temperature, i.e. 25 °C, more preferably having a melting point of 35° C to 120 °C, more preferably of 45 to 80 °C.

Further, waxy compounds as used herein preferably consist of or comprise hydrophobic compounds that are

malleable at 25 °C. Waxy compounds can comprise esters of fatty acids and $C_6$-$C_{30}$, preferably $C_8$-$C_{18}$ alcohols, such as myricyl palmitate or cetyl palmitate. Examples of waxy compounds are natural waxes such as beeswax, lanolin, Carnauba wax containing myricyl cerotate, castor wax, candelilla wax, ouricury wax, sugar cane wax, retamo wax and tallow. Waxy compounds can also comprise mono-, di- and triesters of glycerin and fatty acids such as behenic, oleic, stearic, palmitoleic, arachidic, palmitic, lauric, myristic and ricinoleic acid, as well as phospholipids. Di- and triesters of glycerin and fatty acids can comprise up to two and three different fatty acids, respectively.

In a preferred embodiment, the moisture protective agent has a saponification value of from 10 to 250, preferably from 50 to 220, more preferably from 100 to 200, still more preferably from 125 to 185. The saponification value can be measured according to ASTM D5558.

In a preferred embodiment, the moisture protective agent is selected from the group consisting of hydrogenated vegetable oil, castor oil, palmitol stearate, glyceryl palmitostearate and glyceryl behenate.

In a most preferred embodiment, the moisture protective agent is glyceryl behenate.

"Glyceryl behenate" usually is a mixture of glyceryl esters of behenic acid made from glycerin and behenic acid. Preferably, glyceryl behenate as used in the present invention comprises 10 to 20 percent monoglyceride, 47 to 59 percent diglyceride, 26 to 38 percent triglyceride, and not more than 2.5 percent free behenic acid. Further, glyceryl behenate as used in the present invention preferably has an acid value of not more than 4, as measured according to ASTM D 974. Preferably glyceryl behenate as used in the present invention has a saponification value of from 145 to 165, as measured according to ASTM D5558. Preferably, glyceryl behenate as used in the present invention has an iodine number of not more than 3, as measured according to ASTM D 5554.

In a preferred embodiment, magnesium stearate is not used as moisture protective agent.

The moisture protective agent comprised in the dosage form of the present invention can preferably have an average particle size X90 of 70 to 130 μm, more preferably 80 to 120 μm, most preferably 90 to 110 μm.

The moisture protective agent comprised in the dosage form of the present invention can preferably have an average particle size X50 of 30 to 80 μm, more preferably 40 to 70 μm, most preferably 50 to 60 μm.

The moisture protective agent comprised in the dosage form of the present invention can preferably have an average particle size X10 of 5 to 30 μm, more preferably 10 to 25 μm, most preferably 15 to 20 μm.

The moisture protective agent comprised in the dosage form of the present invention can preferably have a ratio X10/X90 of 0.10 to 0.25, more preferably 0.14 to 0.20, most preferably 0.15-0.18.

The moisture protective agent is preferably provided in an amount of 1 to 8 wt.%, preferably 1.5 to 6 wt.%, more preferably 1.5 to 4 wt.%, in particular, 1.3 to 3 wt.%, based on the total weight of the dosage form.

Further, the moisture protective agent is preferably provided in the dosage form in an amount of 0.5 to 8 mg, preferably 0.75 to 5.0 mg, more preferably 1.0 to 3.0 mg, in particular 1.25 to 2.6 mg.

In one preferred embodiment, the moisture protective agent is glyceryl behenate and is provided in the dosage form in an amount of 0.5 to 8 mg, preferably 0.75 to 5.0 mg, more preferably 1.0 to 3.0 mg, in particular 1.25 to 2.6 mg.

In one embodiment of the invention, the ratio (w/w) of siponimod to moisture protective agent in the pre-blending step ii) is 1:8 to 3:1, preferably 1:3.5 to 2:1, most preferably 1:2 to 1.4:1.

In one embodiment of the invention, siponimod is pre-blended in step ii) with a moisture protective agent and a filler.

Fillers, which are sometimes also called "bulking agents" or "diluents", add volume and/or mass to a drug substance, thereby facilitating precise metering and handling in the preparation of dosage forms. Fillers typically also fill out the size of a tablet or capsule, making it practical to produce and convenient for the consumer to use.

A good filler should typically be inert, compatible with the other components of the formulation, non-hygroscopic, relatively cheap, compactible, and, preferably, tasteless or with a pleasant tasting.

In a preferred embodiment, the filler is referred to as a single filler or to a mixture of several fillers.

Examples of fillers are plant cellulose (pure plant filler), hydroxypropyl cellulose, dibasic calcium phosphate, lactose, sucrose, glucose, mannitol, sorbitol, calcium carbonate, and magnesium stearate.

In an embodiment, the filler is selected from calcium phosphate, calcium hydrogen phosphate, calcium dihydrogen phosphate, calcium carbonate, magnesium carbonate, magnesium aluminosilicates, sugar alcohols, such as mannitol, maltitol, isomalt, sorbitol, xylitol, threitol and erythritol, a triglyceride, such as hydrogenated vegetable oil, mucilage such as carrageenan, agar and pectin, a monosaccharide such as arabinose, xylose, glucose, mannose, galactose, a disaccharide, such as isomaltose, maltose, lactose, sucrose, an oligosaccharide, such as raffinose, oligofructose, cyclodextrins, maltodextrin, a polysaccharide, such as starch, such as corn starch, glycogen and cellulose, such as microcrystalline cellulose, and mixtures thereof.

Preferably, the filler is selected from calcium phosphate, calcium hydrogen phosphate, mannitol, xylose, glucose, galactose maltose, lactose, sucrose, maltodextrin, starch microcrystalline cellulose and a mixture thereof.

In a further preferred embodiment, the filler can preferably be selected from microcrystalline cellulose, glucose, lactose, mannitol and starch and mixtures thereof. More preferably the filler is lactose, microcrystalline cellulose or a mixture thereof.

In one embodiment, lactose is used as filler. Preferably, the lactose used as filler has an X50 value in the range of 70 to 130 μm, preferably 80 to 120 μm, more preferably 90 to 110 μ

If a filler is present in step ii), it is preferred that the filler is provided in step ii) in an amount of 1 to 20 wt.%, preferably 2 to 17 wt.%, more preferably 3 to 12 wt.%, based on the total weight of the dosage form.

In one embodiment of the invention, the ratio (w/w) of filler/siponimod to moisture protective agent in step ii) is 5:1 to 2:1, preferably 4.5:1 to 2.5:1, most preferably 4:1 to 3:1.

The pre-blending can be carried out with mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany) or a high shear mixer. Blending can be carried out e.g. for 1 minute to 30 minutes, preferably for 2 minutes to 10 minutes. If a diffusion mixer is used, blending can be carried out at e.g. 15 to 50 rpm, preferably 15 to 30 rpm.

The pre-blending is typically carried out at room temperature, i.e. 25°C.

In an alternative embodiment, the pre-blending can be conducted such that the siponimod ii) is mixed with a first part of the moisture-protective agent or a mixture of the moisture-protective agent and pharmaceutical excipient in a mixing device, for example in a high shear or diffusion mixer. After this first mixing step, a second part of the moisture-protective agent or a mixture of the moisture-protective agent and pharmaceutical excipient can be added, which is followed by a second mixing step. This procedure can be repeated until the last part of the moisture-protective agent or a mixture of the moisture-protective agent and pharmaceutical excipient is used, preferably one to five times.

After the pre-blending in step ii), the resulting mixture, can be sieved before being blended with one or more pharmaceutical preferably comprising agglomerates of siponimod and moisture protective agent, excipients in step iii). In a preferred embodiment, the sieve has a mesh size of 100 to 1000 μm, preferably of 200 to 800 μm.

In step iii), the mixture of step ii) is blended with one or more pharmaceutical excipients. The one or more pharmaceutical excipients are preferably selected from the group consisting of fillers, glidants, disintegrants, binders and lubricants.

Fillers are defined as above.

In an embodiment, the filler is selected from calcium phosphate, calcium hydrogen phosphate, calcium dihydrogen phosphate, calcium carbonate, magnesium carbonate, magnesium aluminosilicates, sugar alcohols, such as mannitol, maltitol, isomalt, sorbitol, xylitol, threitol and erythritol, a triglyceride, such as hydrogenated vegetable oil, mucilage such as carrageenan, agar and pectin, a monosaccharide such as arabinose, xylose, glucose, mannose, galactose, a disaccharide, such as isomaltose, maltose, lactose, sucrose, an oligosaccharide, such as raffinose, oligofructose, cyclodextrins, maltodextrin, a polysaccharide, such as starch, such as corn starch, glycogen and cellulose, such as microcrystalline cellulose, and mixtures thereof.

Preferably, the filler is selected from calcium phosphate, calcium hydrogen phosphate, mannitol, xylose, glucose, galactose maltose, lactose, sucrose, maltodextrin, starch microcrystalline cellulose

In a further preferred embodiment, the filler can preferably be selected from microcrystalline cellulose, glucose, lactose, mannitol and starch and mixtures thereof.

More preferably the filler is lactose, microcrystalline cellulose or a mixture thereof.

In a particularly preferred embodiment, the filler is a mixture of microcrystalline cellulose and lactose.

Glidants can be used to improve the flowability. A preferred glidant is colloidal silica, preferably having a specific surface area of 80 to 250 $m^2/g$, determined according to Pharm. Eur 6.0, 2.9.26 by gas adsorption. Preferably, the glidant can be present in an amount of 0 to 3 wt.%, more preferably of 0.1 to 2.7 wt.%, in particular of 0.3 to 2.5 wt.% based on the total weight of the dosage form. In one embodiment, the glidant can be present in the dosage form in an amount of 0 to 6 mg, preferably 0.1 to 5.0 mg, more preferably 0.2 to 4.0 mg, in particular, 0.5 to 3.0 mg.

Disintegrants are substances which can enhance the ability of the intermediate to break into smaller fragments when in contact with a liquid, preferably water. Preferred disintegrants are guar galactomannan, sodium carboxymethyl starch (croscarmellose sodium), cross-linked polyvinylpyrrolidone (crospovidone), sodium carboxymethyl glycolate, sodium bicarbonate or mixtures thereof, most preferred is cross-linked polyvinylpyrrolidone. Disintegrants can preferably be present in amounts of 0 to 10 wt.%, preferably of 0.1 to 8 wt.%, in particular of 0.2 to 6 wt.%, based on the total weight of the dosage form. In one embodiment, the disintegrant can be provided in the dosage form in an amount of 0 to 15 mg, preferably 0.5 to 12 mg, more preferably 1 to 8.5 mg, in particular 1.5 to 6.5 mg.

Binders are substances that ensure that granules or tablets can be formed with the required mechanical strength. Binders can be, for example, saccharose, gelatine, polyvinylpyrrolidone, starch, cellulose derivatives such as hydroxylpropyl cellulose, hydroxyethyl cellulose, hydroxypropyl methylcellulose (HPMC). Binders can preferably be present in amounts of 0 up to 10 wt.%, preferably of 2 to 8 wt.%, in particular of 2.5 to 6.5 wt.%, based on the total weight of the dosage form. In another embodiment of the invention the binder is provided in the dosage form in an amount of 0 to 20 mg, preferably 0.5 to 15 mg, more preferably 1 to 10 mg, in particular 1.5 to 8.5 mg.

In a preferred embodiment no binder is used, in particular, when microcrystalline cellulose is used as filler or part thereof.

Lubricants are generally used in order to reduce sliding friction. In particular, the intention is to reduce the sliding

friction found during tablet pressing between the punch moving up and down in the die and the die wall, on the one hand, and between the edge of the tablet and the die wall, on the other hand. Suitable lubricants are, for example, stearic acid, adipic acid, sodium stearyl fumarate and/or glyceryl behenate, preferably glyceryl behenate. Preferably, lubricants can be present in an amount of 0 to up to 8 wt.%, more preferably of 0.5 to 6 wt.%, in particular of 1 to 4.5 wt.%, based on the total weight of the dosage form. In another embodiment of the invention the lubricant is provided in the dosage form in an amount of 0 to 8 mg, preferably 0.1 to 6 mg, more preferably 0.2 to 4.5 mg, in particular 0.3 to 4.0 mg.

Preferably, no lubricant is added in step iii).

In a preferred embodiment, the mixture of step ii) is blended in step iii) with filler, disintegrant and glidant.

If in step ii) siponimod is pre-blended with moisture-protective agent and pharmaceutical excipient, e.g. a filler, in step iii) the same excipient as in step ii) can be added again to the mixture resulting from step ii). Alternatively, if the at least one pharmaceutical excipient, e.g. the filler, is composed of a mixture of a first and a second compound, e.g. lactose and microcrystalline cellulose, the first compound of the mixture can be added in step ii), the second compound in step iii), optionally together with other pharmaceutical excipients. Yet further, if the at least one pharmaceutical excipient, e.g. the filler, is composed of a mixture of a first and a second compound, e.g. lactose and microcrystalline cellulose, a first part of the first compound of the mixture can be added in step ii), a second part of the first compound and the second compound can be added in step iii), optionally together with other pharmaceutical excipients.

It is preferred that the filler is provided in step iii) in an amount of 29 to 97.7 wt.%, preferably 40.3 to 92.5 wt.%, more preferably 62.8 to 90.5 wt.%, based on the total weight of the dosage form.

In one embodiment, the total amount of filler in the dosage form is 17.8 mg to 84.3 mg, preferably 31.8 mg to 82.05 mg, more preferably 52.4 mg to 80.6 mg, in particular 58.1 mg to 78.25 mg.

It is noted that due to the nature of pharmaceutical excipients it cannot be excluded that a certain compound meets the requirements of more than one of the pharmaceutical excipients disclosed above.

In the context of this invention, for the sake of clarity, a substance which is used as a particular excipient preferably is not simultaneously also used as a further pharmaceutical excipient. For example, microcrystalline cellulose - if used as filler - is not also used as, for example, disintegrant, even though microcrystalline cellulose also exhibits a certain disintegrating effect. To this general rule one exception applies: an excipient being used as moisture protective agent may also be used as lubricant (preferably in step iiia), see below.

The blending of the one or more pharmaceutical excipient(s) with the mixture after step ii) can be carried out with conventional mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany). Blending can be carried out e.g. for 1 minute to 40 minutes, preferably for 5 minutes to 25 minutes at e.g. 3 to 30 rpm, preferably 5 to 15 rpm.

The blending is typically carried out at room temperature, i.e. 25°C.

After the blending in step iii), the resulting mixture can be sieved. In a preferred embodiment, the sieve has a mesh size of 100 to 1000 μm, preferably of 200 to 800 μm.

In a further embodiment of the invention, the mixture obtained after step iii) is blended in a step iiia) with at least one further pharmaceutical excipient. The at least one pharmaceutical excipient is preferably selected from the group consisting of lubricants, binders, glidants, disintegrants and fillers, which are defined according to the above remarks regarding step iii). In a most preferred embodiment, the at least one pharmaceutical excipient blended in step iiia) is a lubricant.

In one embodiment of the invention, the lubricant used in step iiia) is selected from the group comprising stearic acid, adipic acid, sodium stearyl fumarate and glyceryl behenate. In a preferred embodiment, the lubricant is glyceryl behenate.

Preferably, lubricants can be present in an amount of 0 to up to 8 wt.%, more preferably of 0.5 to 6 wt.%, in particular of 1 to 4.5 wt.%, based on the total weight of the dosage form. In another embodiment of the invention the lubricant is provided in the dosage form in an amount of 0 to 8 mg, preferably 0.1 to 6 mg, more preferably 0.2 to 4.5 mg, in particular 0.3 to 4.0 mg.

In a preferred embodiment, the at least one pharmaceutical excipient from optional step iiia) can be sieved by using a screening mill before being blended with the mixture obtained from step iii). In a preferred embodiment, the sieve has a mesh size of 100 to 1000 μm, preferably of 200 to 800 μm.

The blending of the at least one pharmaceutical excipient in optional step iiia) with the mixture obtained after step iii) can be carried out with conventional mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany). Blending can be carried out e.g. for 1 minute to 30 minutes, preferably for 2 minutes to 10 minutes at e.g. 3 to 30 rpm, preferably 5 to 15 rpm.

In an embodiment of the invention, after the blending in step iiia), the resulting mixture can be sieved. In a preferred embodiment, the sieve has a mesh size of 100 to 1000 μm, preferably of 200 to 800 μm.

In step iv), the mixture from step iii) or iiia) can be further processed into a dosage form. In one preferred

embodiment, the step iv) of processing the mixture from step iii) or iiia) in a dosage form can include compressing the respective mixture into tablets.
The compression of the mixture from step iii) or iiia) can be preferably a direct compression. This direct compression step can preferably be carried out on a rotary press, for example on a Fette® 102i (Fette GmbH, Germany) or a Riva® piccola (Riva, Argentina). More preferably the tablets are formed on a Korsch PH250 or Korsch XL400 (Korsch AG, Germany). The compaction force can range from 1 to 60 kN, preferably from 2 to 30 kN, more preferably from 3 to 15 kN.

In optional step v), the dosage form, preferably the tablet, is film-coated. The above-mentioned amounts of siponimod, moisture protective agent and excipients, however, relate to the uncoated dosage form.

For film-coating, macromolecular substances are preferably used, such as modified celluloses, polymethacrylates, polyvinylpyrrolidone, polyvinyl acetate phthalate and/or shellack. In a preferred embodiment the coating can have a thickness of 2 to 80 μm, more preferably 5 to 50 μm.

As mentioned above, in a preferred embodiment the moisture-protective agent and the lubricant can be the same compound, while having two different functions. The compound added to siponimod in step ii) functions as moisture-protective agent, i.e. it can adhere to the siponimod particles, while the same compound further added in step iii) and/or iiia) may functions as lubricant. When the moisture-protective agent and the lubricant are the same compound, the combined amount of lubricant and moisture-protective agent in the dosage form is 1 to 16 wt.%, preferably 2 to 12 wt.%, more preferably 3.0 to 6.0 wt.%, based on the total weight of the dosage form. In a preferred embodiment, glyceryl behenate is used in step ii) as moisture-protective agent and in step iii) and/or iiia) as lubricant. In a preferred embodiment, when glyceryl behenate is used as moisture-protective agent and as lubricant, the dosage form of the present invention comprises 0.5 to 16 mg, preferably 1 to 12 mg, more preferably 2 to 8 mg, especially 2.5 to 6 mg glyceryl behenate.

In a preferred embodiment, the dosage form of the present invention can preferably comprise the following amounts of components:

- 0.2 to 12 wt.%, preferably 1.3 to 12 wt.%, more preferably 1.3 to 3.0 wt.%, especially 2.0 to 2.7 wt.% siponimod,
- 1 to 8 wt.%, preferably 1.5 to 6 wt.%, more preferably 1.5 to 4 wt.%, in particular 1.5 to 3 wt.% moisture-protective agent,
- 0 to 8 wt.%, preferably 0.5 to 6 wt.%, in particular of 1 to 4.5 wt.% lubricant,
- 0 to 10 wt.%, preferably of 2 to 8 wt.%, in particular of 2.5 to 6.5 wt.% binder,
- 0 to 3 wt.%, preferably 0.1 to 2.7 wt.%, in particular 0.3 to 2.5 wt.% glidant,
- 0 to 10 wt.%, more preferably 0.1 to 8 wt.%, in particular of 0.2 to 6 wt.% disintegrant,
- 49 to 98.7 wt.%, more preferably 57.3 to 94.5 wt.%, in particular 74.8 to 93.5 wt.% filler,

wherein the wt.% are based on the total weight of the dosage form.

In a preferred embodiment, the dosage form of the present invention can preferably comprise the following amounts of components:

- 1.0 to 2.7 wt.% siponimod,
- 1.0 to 2.5 wt.% moisture protective agent,
- 2.5 to 3.5 wt.% lubricant,
- 0 wt.% binder,
- 0.3 to 0.6 wt.% glidant,
- 5.2 to 6.2 wt.% disintegrant,
- 84.5 to 90.0 wt.% filler,

wherein the wt.% are based on the total weight of the dosage form.

In a preferred embodiment, the dosage from of the present invention can preferably comprise the following amounts of components:

- 0.2 to 10 mg, preferably 1.0 to 10 mg, more preferably 1.0 to 2.6 mg, in particular 1.7 to 2.3 mg siponimod,
- 0.5 to 8 mg, preferably 0.75 to 5.0 mg, more preferably 1.0 to 3.0 mg, in particular, 1.25 to 2.6 mg moisture protective agent,
- 0 to 8 mg, preferably 0.1 to 6.0 mg, more preferably 0.2 to 4.5 mg, in particular, 0.3 to 4.0 mg lubricant,
- 0 to 20 mg, preferably 0.5 to 15 mg; more preferably 1 to 10 mg, in particular, 1.5 to 8.5 mg binder,
- 0 to 6 mg, preferably 0.1 to 5.0 mg, more preferably 0.2 to 4.0 mg, in particular, 0.5 to 3.0 mg glidant,
- 0 to 15 mg, preferably 0.5 to 12 mg, more preferably 1 to 8.5 mg, in particular 1.5 to 6.5 mg disintegrant,
- 17.8 to 84.3 mg, preferably 31.8 to 82.05 mg, more preferably 52.4 to 80.6 mg, in particular, 58.1 to 78.25 mg filler,

In a preferred embodiment, the dosage from of the present invention can preferably comprise the following

amounts of components:

- 1.7 to 2.3 mg siponimod,
- 0.8 to 5.0 mg moisture protective agent,
- 2.0 to 2.7 mg lubricant,
- 0 mg binder,
- 0.25 to 0.55 mg glidant,
- 4.5 to 5.9 mg disintegrant,
- 68.55 to 75.75 mg filler.

In a further embodiment, the dosage form of the present invention can preferably comprise the following amounts of components:

0.2 to 10 mg siponimod,
0.5 to 8 mg moisture-protective agent,
0 to 8 mg lubricant,
0 to 20 mg binder,
0 to 6 mg glidant,
0 to 15 mg disintegrant, and
17.8 to 84.3 mg filler.

In a preferred embodiment, the dosage from of the present invention can preferably comprise the following amounts of components:

- 1.7 to 2.3 mg siponimod,
- 0.8 to 5.0 mg glyceryl behenate, preferably as moisture protective agent,
- 2.0 to 2.7 mg glyceryl behenate, preferably as lubricant,
- 0 mg binder,
- 0.25 to 0.55 mg Aerosil® 200, preferably as glidant,
- 4.5 to 5.9 mg crospovidone, preferably as disintegrant,
- 68.55 to 75.75 mg lactose and microcrystalline cellulose (=MCC), preferably as filler, wherein preferably lactose and MCC are present in a mixture having a ratio of lactose/MCC of 1:1 to 10:1, more preferably of 4:1 to 5:1.

In a preferred embodiment, the dosage form of the present invention has a residual water content from 0.1 to less than 8 wt.%, preferably from 1 to 7 wt.%, more preferably from 3 to 6 wt.-%. The residual water content can be determined according to the Karl Fischer Method as described in Ph. Eur. 6th edition, section 2.5.12. The determination is done by coulometry, whereby a coulometer is used at 105 °C, preferably a Metrohm 831 KF Coulometer, including a titration cell without diaphragm. Usually, a sample of 350 mg of the dosage form is analyzed. The water content can be achieved by choosing appropriate ingredients and/or drying.

In a preferred embodiment, the dosage form is a tablet.

The tablet preferably has a content uniformity of 90 to 110 %, preferably 95 to 105 %, more preferably 96 to 104 %, especially 97 to 103 % of the average content. The content uniformity is determined in accordance with Ph. Eur., 7.0. This means that each of twenty individual samples of the dosage form has a siponimod content of between 90 % and 110 %, preferably 95 % to 105 %, even more preferably 98% to 102 % of the average content of those twenty individual samples.

In a further embodiment, the in-vitro release of siponimod of the dosage form according to the invention is not less than 80 % after 30 min (immediate release, IR). This means that the release profile of the dosage forms of the invention according to USP app. II (paddle, 500 ml for dosage strength ≤ 0.25 mg, 900 ml for dosage strength >0.25 mg, phosphate buffer + 0.1% (m/v) Tween® 80, 60 rpm, 37°C, determination by HPLC detection) after 30 minutes preferably indicates a content release of not less than 80%, preferably more than 90%, and preferably up to 95 % or more preferably up to 100 %.

Tween® 80 has the name polyoxyethylene(20) sorbitan monooleate of the formula

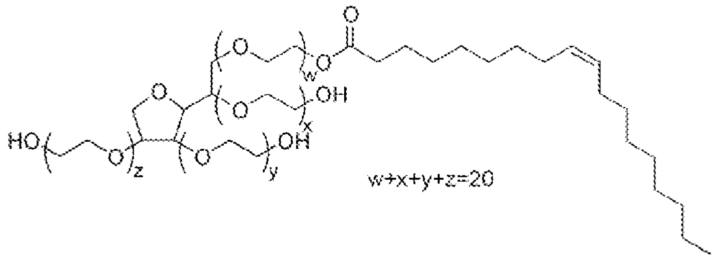

and has a density at 25°C of around 1.06-1.09 g/mL, a viscosity at 25°C of 300-500 mPa·s and a HLB-value (hydrophilic-lipophilic balance value) of 15.0, determined by the Griffin's method.

In an embodiment of the dosage form of the present invention 0 to 4 wt.% of siponimod is decomposed after 6 months at 25°C at a humidity of 60%.

A further subject of the invention is a process for producing a dosage form comprising the steps

i) providing siponimod,
ii) pre-blending the compounds of step i) with moisture-protective agent and optionally pharmaceutical excipient,
iii) blending the mixture of step ii) with pharmaceutical excipient(s),
iv) processing the mixture of step iii) to a dosage form,
v) optionally film-coating the dosage form.

Generally, all explanations concerning preferred embodiments of the dosage form of the present invention, e.g. relating to the components siponimod, moisture-protective agent and further pharmaceutical excipients and their amounts etc., also apply to the process of the present invention. For the sake of completeness, some preferred embodiments of the process are mentioned again below.

In step i) according to the present invention, siponimod is provided. As described above, siponimod can be provided as a free base, as a pharmaceutically acceptable salt, polymorph, solvate, hydrate or prodrug thereof. In a preferred embodiment it is provided as siponimod hemifumarate.

In step ii) of the present invention, siponimod is pre-blended with moisture protective agent. The moisture protective agent has the objective to coat the siponimod particles and protect them from moisture during the dosage form formation process.

In another embodiment, siponimod is pre-blended in step ii) with a moisture-protective agent and a pharmaceutical excipient as disclosed above, preferably a filler.

Preferably, the pre-blending step ii) is carried out at room temperature, i.e. 25°C.

The pre-blending can be carried out with conventional mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany). Blending can be carried out e.g. for 1 minute to 30 minutes, preferably for 2 minutes to less than 10 minutes at e.g. 15 to 50 rpm, preferably 15 to 30 rpm.

In an alternative embodiment, the mixing can be conducted such that the siponimod in step ii) is mixed with a first part of the moisture-protective agent or a mixture of the moisture-protective agent and a pharmaceutical excipient in a mixing device, for example in a high shear or tumbler mixer. After this first mixing step, a second part of the moisture-protective agent or a mixture of the moisture-protective agent and a pharmaceutical excipient can be added, which is followed by a second mixing step. This procedure can be repeated until the last part of the moisture-protective agent or a mixture of the moisture-protective agent and a pharmaceutical excipient is used, preferably one to five times.

After the pre-blending in step ii), the resulting mixture can be sieved before being blended with one or more pharmaceutical excipients in step iii). In a preferred embodiment, the sieve has a mesh size of 100 to 1000 μm, preferably of 200 to 800 μm.

In step iii), the mixture of step ii) is blended with one or more pharmaceutical excipients. The one or more pharmaceutical excipients are preferably selected from the group consisting of lubricants, binders, glidants, disintegrants and fillers.

Preferably, the iii) is carried out at room temperature, i.e. 25°C.

The blending of the one or more pharmaceutical excipients with the mixture after step ii) can be carried out with conventional mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany).

Blending can be carried out e.g. for 1 minute to 40 minutes, preferably for 5 minutes to 25 minutes at e.g. 3 to 30 rpm, preferably 5 to 15 rpm.

In an alternative embodiment, the mixing can be conducted such that the mixture of step ii) is mixed with a first

part of the at least one pharmaceutical excipient in a mixing device, for example in a high shear or tumbler mixer. After this first mixing step, a second part of the at least one pharmaceutical excipient can be added, which is followed by a second mixing step. This procedure can be repeated until the last part of the at least one pharmaceutical excipient is used, preferably one to five times.

In a further alternative embodiment, the mixing can be conducted such that the mixture of step ii) is mixed with a first pharmaceutical excipient of the at least one pharmaceutical excipient in a mixing device, for example in a high shear or tumbler mixer. After this first mixing step, a second pharmaceutical excipient of the at least one pharmaceutical excipient is added, which is followed by a second mixing step. Hence, the number of mixing steps corresponds to the number of pharmaceutical excipients of step iii).

If in step ii) siponimod is pre-blended with a moisture-protective agent and a pharmaceutical excipient, in step iii) the same excipient as in step ii) can be added again to the mixture resulting from step ii). Alternatively, if the at least one pharmaceutical excipient, e.g. the filler, is composed of a mixture of a first and a second compound, e.g. lactose and microcrystalline cellulose, the first compound of the mixture can be added in step ii), the second compound in step iii), eventually together with other pharmaceutical excipients. Yet further, if the at least one pharmaceutical excipient, e.g. the filler, is composed of a mixture of a first and a second compound, e.g. lactose and microcrystalline cellulose, a first part of the first compound of the mixture can be added in step ii), a second part of the first compound and the second compound can be added in step iii), optionally together with other pharmaceutical excipients.

After the blending in step iii), the resulting mixture can be sieved. In a preferred embodiment, the sieve has a mesh size of 100 to 1000 μm, preferably of 200 to 800 μm.

In an embodiment of the invention, the mixture obtained after step iii) is blended in an optional step iiia) with at least one further pharmaceutical excipients. The at least one pharmaceutical excipient is preferably selected from the group consisting of lubricants, binders, glidants, disintegrants and fillers, which are defined according to the above remarks regarding step iii).

In a preferred embodiment, the at least one pharmaceutical excipient from optional step iiia) can be sieved by using a screening mill before being blended with the mixture obtained from step iii). In a preferred embodiment, the sieve has a mesh size of 100 to 1000 μm, preferably of 200 to 800 μm.

The blending of the at least one pharmaceutical excipient in optional step iiia) with the mixture obtained after step iii) can be carried out with conventional mixing devices, e.g. in a diffusion mixer like Turbula® T10B (Willy A. Bachofen AG Maschinenfabrik, Muttenz, Switzerland) or Bohle PM400S (L.B. Bohle Maschinen + Verfahren GmbH, Ennigerloh, Germany). Blending can be carried out e.g. for 1 minute to 30 minutes, preferably for 2 minutes to 10 minutes at e.g. 3 to 30 rpm, preferably 5 to 15 rpm.

In an alternative embodiment, the mixing can be conducted such that the mixture of step iii) is mixed with a first part of the at least one pharmaceutical excipient in a mixing device, for example in a high shear or tumbler mixer. After this first mixing step, a second part of the at least one pharmaceutical excipient can be added, which is followed by a second mixing step. This procedure can be repeated until the last part of the at least one pharmaceutical excipient is used, preferably one to five times.

In a further alternative embodiment, the mixing can be conducted such that the mixture of step iii) is mixed with a first pharmaceutical excipient of the at least one pharmaceutical excipient in a mixing device, for example in a high shear or tumbler mixer. After this first mixing step, a second pharmaceutical excipient of the at least one pharmaceutical excipient is added, which is followed by a second mixing step. Hence, the number of mixing steps corresponds to the number of pharmaceutical excipients of step iiia).

In a preferred embodiment, one pharmaceutical excipient is mixed in step iiia).

In an embodiment of the invention, after the blending in step iiia), the resulting mixture can be sieved. In a preferred embodiment, the sieve has a mesh size of 100 to 1000 μm, preferably of 200 to 800 μm.

In step iv), the mixture from step iii) or iiia) can be further processed into a dosage form. In one preferred embodiment, the step iv) of processing the mixture from step iii) or iiia) in a dosage form can include compressing the respective mixture into tablets.

The compression of the mixture from step iii) or iiia) can be preferably a direct compression. This direct compression step can preferably be carried out on a rotary press, for example on a Fette® 102i (Fette GmbH, Germany) or a Riva® piccola (Riva, Argentina). More preferably the tablets are formed on a Korsch PH250 or Korsch XL400.

If a rotary press is applied, the main compaction force can range from 1 to 60 kN, preferably from 2 to 30 kN, more preferably form 3 to 15 kN.

In optional step v), the dosage form, preferably the tablet, is film-coated. For this purpose, standard methods of film-coating tablets may be employed. The above-mentioned amounts of siponimod, moisture protective agent and excipients, however, relate to the uncoated tablet.

For film-coating, macromolecular substances are preferably used, such as modified celluloses, polymethacrylates, polyvinylpyrrolidone, polyvinyl acetate phthalate and/or shellack.

## Examples

Example 1 - Process Blending

In order to obtain a final mixture ready to be processed to a dosage form, e.g. a tablet, siponimod hemifumarate having a X90 value of 18 μm is blended with different excipients according to the flow diagram of Figure 1. Therefore, siponimod hemifumarate is pre-blended in step 1 with a mixture of glyceryl behenate as moisture protective agent and spray-dried lactose as filler. The pre-blending is carried out in a diffusion mixer Bohle PM400S (L.B. Bohle Maschinen + Verfahren GmbH, Ennigerloh, Germany) for 10 min at 10 rpm. The mixture of step 1 is then sieved in step 2 using a screening mill having a mesh size of 800 μm. The sieved mixture is then blended in step 3 with further spray-dried lactose as filler, Aerosil as glidant, polyvinylpolypyrrolidon XL (crospovidone) as disintegrant and microcrystalline cellulose GR as filler in a diffusion mixer Bohle PM400S for 5 min at 10 rpm. The resulting mixture is again sieved in step 4 using an oscillating screening mill Frewitt GLA ORV having a mesh size of 800 μm and mixed in step 5 in a diffusion mixer Bohle PM400S for 25 min at 10 rpm. In step 6 glyceryl behenate as lubricant, which has been sieved using an oscillating screening mill Frewitt GLA ORV having a mesh size of 800 μm, is added to the mixture of step 5 and mixed in step 7 in a diffusion mixer Bohle PM400S for 10 min at 10 rpm, resulting in the final dosage form mixture.

The final dosage mixture resulted from the blending process is then processed into a dosage form, preferably a tablet. The tablets are formed using a rotary tablet press, a Korsch PH 250 or Korsch XL400 with a compression force of 6 kN. The tablets are then de-dusted with a Kramer deduster (Kramer AG, Switzerland) and finally coated by a perforated pan coater Glatt Coater GC 750 (Glatt GmbH, Germany).

Example 2 - Manufacturing Process of Siponimod Film Coated Tablets

Following the process of Example 1, film-coated tablets with the composition per tablet according to Tables 1 to 4 can be prepared.

**Table 1:**

| Component | Composition per unit [%] | Composition per unit [mg/unit] |
|---|---|---|
| Siponimod hemifumarate* | 0.33 | 0.278 |
| (X90 = 18 μm) | | |
| Lactose - preblending step 1 | 7.32 | 6.220 |
| Lactose - step 3 | 65.85 | 55.977 |
| Total Lactose | 73.17 | 62.197 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |
| * The salt factor is 1.112 | | |

**Table 2:**

| Component | Composition per unit [%] | Composition per unit [mg/unit] |
|---|---|---|
| Siponimod hemifumarate* (X90 = 18 μm) | 0.65 | 0.556 |
| Lactose - preblending step 1 | 7.29 | 6.192 |

(continued)

| Component | Composition per unit [%] | Composition per unit [mg/unit] |
|---|---|---|
| Lactose - step 3 | 65.56 | 55.727 |
| Total Lactose | 72.85 | 61.919 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |
| * The salt factor is 1.112 | | |

**Table 3:**

| Component | Composition per unit [%] | Composition per unit [mg/unit] |
|---|---|---|
| Siponimod hemifumarate* (X90 = 18 μm) | 1.31 | 1.112 |
| Lactose - preblending step 1 | 7.22 | 6.136 |
| Lactose - step 3 | 64.97 | 55.227 |
| Total Lactose | 72.19 | 61.363 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |
| * The salt factor is 1.112 | | |

**Table 4:**

| Component | Composition per unit [%] | Composition per unit [mg/unit] |
|---|---|---|
| Siponimod hemifumarate* (X90 = 18 μm) | 2.62 | 2.224 |
| Lactose - preblending step 1 | 7.09 | 6.025 |
| Lactose - step 3 | 63.79 | 54.226 |
| Total Lactose | 70.88 | 60.251 |
| Microcryst. cellulose | 15.0 | 12.750 |

(continued)

| Component | Composition per unit [%] | Composition per unit [mg/unit] |
|---|---|---|
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |
| * The salt factor is 1.112 | | |

Example 3 - Process Blending

A process according to Example 1 has been carried out, wherein lactose and polyvinylpolypyrrolidon XL (crospovidone) have been replaced by mannitol and croscarmellose sodium, respectively.

Example 4 - Manufacturing Process of Siponimod 2 mg Film Coated Tablet

Following the process of Example 3, film-coated tablets with the composition per tablet according to Table 5 can be prepared.

**Table 5:**

| Component | Composition per unit [%] | Composition per unit [mg/unit] |
|---|---|---|
| Siponimod hemifumarate* (X90 = 18 μm) | 2.62 | 2.224 |
| Mannitol - preblending step 1 | 7.09 | 6.025 |
| Mannitol - step 3 | 63.79 | 54.226 |
| Total Mannitol | 70.88 | 60.251 |
| Microcryst. cellulose | 15.0 | 12.750 |
| Croscarmellose-N a | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |
| * The salt factor is 1.112 | | |

Example 5 - Stability Tests

Two different tablets with the composition according to Table 6 have been produced. The tablet of Example 5-1 has been produced according to Example 1 (i.e. comprising a pre-blending with glyceryl behenate as moisture protective agent), the tablet of Comparative Example 5-2 has been produced according to Example 1, however no pre-blending step has been carried out. That means that for the production of the tablet of Comparative Example 5-2, glyceryl

behenate has been added only in step 6 as lubricant to the mixture of siponimod and excipients. The siponimod hemifumarate particles used for the tablet formation process had a X90 value of 6 μm.

**Table 6:**

| | Ex. 5-1 | Comp. Ex. 5-2 |
|---|---|---|
| Component | Composition per unit [mg/unit] | Composition per unit [mg/unit] |
| Siponimod hemifumarate* (X90 = 6 μm) | 0.278 | 0.278 |
| Lactose | 58.797 | 58.797 |
| Microcryst. cellulose | 17.0 | 17.0 |
| Polyvinylpolypyrrolidon XL | 2.55 | 2.55 |
| Aerosil 200 | 0.425 | 0.425 |
| Glyceryl behenate | 5.95 | 5.95 |
| *Total core tablet* | *85.000 mg* | *85.000 mg* |
| Pre-blending with glyceryl behenate | yes | no |
| * The salt factor is 1.112 | | |

The so obtained tablets were analyzed directly after formation (T0) in view of siponimod content/purity. Then, the tablets were stored for four weeks at 40°C and 75% humidity. An analysis of the tablets followed the storage (T4w). The results are summarized in Table 7 below.

**Table 7:**

| | Assay (%) T0-T4w |
|---|---|
| Ex. 5-1 | -2.3 |
| Comp. Ex. 5-2 | - 4.6 |

The results clearly show that the tablet comprising agglomerations of moisture protective agent and siponimod, shows less degradation and thus higher stability after four weeks.

The use of agglomerates comprising siponimod and moisture protective agent enables the production of a dosage form having advantageous shelf life.

Example 6 - Stability Test

Tablets with the composition according to Table 8 have been produced. The tablets of Example 6 have been produced according to Example 1, i.e. comprising a pre-blending with glyceryl behenate as moisture protective agent. The siponimod hemifumarate particles used for the tablet formation process had a X90 value of 49 μm.

**Table 8:**

| | Ex. 6 |
|---|---|
| Component | Composition per unit [mg/unit] |
| Siponimod hemifumarate* (X90 = 49 μm) | 0.278 |
| Lactose | 62.197 |
| Microcryst. cellulose | 12.75 |
| Polyvinylpolypyrrolidon XL | 5.1 |
| Aerosil 200 | 0.425 |
| Glyceryl behenate | 4.25 |
| *Total core tablet* | *85.000 mg* |

(continued)

| | Ex. 6 |
|---|---|
| Component | Composition per unit [mg/unit] |
| Pre-blending with glyceryl behenate | yes |
| * The salt tactor is 1.112 | |

The so obtained tablets were coated. The film-coated tablets were analyzed directly (T0) in view of siponimod content/purity. Then, the tablets were stored in HDPE bottles with dessicant for six weeks at 40°C and 75% humidity. An analysis of the tablets followed the storage (T4w). The results are summarized in Table 9 below.

**Table 9:**

| | Assay (%) T0-T6w |
|---|---|
| Ex. 6 | -1.6 |

Example 7 - Dissolution Tests

For the dissolution tests, a USP dissolution apparatus 2 (paddle) has been used.

The dissolution conditions are summarized in Table 10 below. The dissolution tests has been carried out according to USP <711> "Dissolution".

**Table 10:**

| | |
|---|---|
| Speed of rotation | 60 ± 2 rpm |
| Test medium | Phosphate buffer pH 6.8 + 0.1% (m/v) Tween 80 |
| Volume of test medium | 500 mL for the 0.25 mg dosage strength 900 mL for the 0.5, 1 and 2 mg dosage strengths |
| Temperature | 37 ± 0.5°C |

The dissolution rates of the siponimod tablets of Example 2 are summarized in Table 11 below.

**Table 11:**

| **Dosage strength** | **Dissolution rate [%] after** | | | | | |
|---|---|---|---|---|---|---|
| | **5min** | **15min** | **30min** | **45min** | **60min** | **75min** |
| 0.25 mg | 34% | 92% | 99% | 100% | 100% | 100% |
| 0.5 mg | 36% | 91% | 98% | 99% | 99% | 99% |
| 1 mg | 37% | 87% | 97% | 99% | 100% | 100% |
| 2mg | 50% | 87% | 96% | 98% | 99% | 100% |

According to the results in Table 11, the in-vitro release of siponimod is an immediate release.

Example 8 - Content Uniformity

The Content Uniformity of the tablets of Example 2 has been determined according to the Ph. Eur. 7.0. The results are summarized in Table 12 below.

**Table 12:**

| **Dosage strength** | **Content uniformity [%]** |
|---|---|
| 0.25 mg | 94.7 - 103.8 |
| 0.5 mg | 97.0 - 101.6 |

(continued)

| Dosage strength | Content uniformity [%] |
|---|---|
| 1 mg | 97.8 - 102.7 |
| 2 mg | 95.3 - 100.4 |

## Claims

**1.** Dosage form obtainable by a process comprising the steps

i) providing siponimod,
ii) pre-blending the compound of step i) with moisture protective agent and optionally pharmaceutical excipient,
iii) blending the mixture of step ii) with pharmaceutical excipient(s),
iv) processing the mixture of step iii) to a dosage form,
v) optionally, film-coating the dosage form.

**2.** Dosage form according to claim 1 containing 0.2 to 12% (w/w) siponimod, 1 to 8% (w/w) of moisture-protective agent and 80 to 98.8 % (w/w) of pharmaceutical excipient(s).

**3.** Dosage form according to claim 1 or 2, wherein siponimod is present in an amount of 0.2 to 10 mg, based on the amount of siponimod in form of the free base.

**4.** Dosage form according to any one of claims 1 to 3, wherein the in-vitro release of siponimod is not less than 80 % after 30 minutes.

**5.** Dosage form according to any one of claims 1 to 4, wherein the ratio of the siponimod particle size X90/X50 is 2 to 5.

**6.** Dosage form according to any one of claims 1 to 5, wherein the moisture-protective agent has a n-octanol/water partition coefficient (*log P*) of 0.1 to 20, preferably 1 to 15.

**7.** Dosage form according to any one of claims 1 to 6, wherein the moisture-protective agent is selected from hydrogenated vegetable oil, castor oil, palmitol stearate, glyceryl palmitostearate and glyceryl behenate.

**8.** Dosage form according to claim 7, wherein the moisture-protective agent is glyceryl behenate.

**9.** Dosage form according to any one of claims 1 to 8, wherein the pharmaceutical excipients are selected from lubricants, binders, glidants, disintegrants and fillers.

**10.** Dosage form according to any one of claims 1 to 9, wherein the lubricant and the moisture-protective agent are the same compound, wherein the combined amount of lubricant and moisture-protective agent is 1 to 16 % (w/w).

**11.** Dosage form according to any one of claims 1 to 10, containing

0.2 to 12 wt.% siponimod,
1 to 8 wt.% moisture-protective agent,
0 to 8 wt.% lubricant,
0 to 10 wt.% binder,
0 to 3 wt. % glidant,
0 to 10 wt.% disintegrant, and
49 to 98.7 wt.% filler, based on the total weight of the dosage form.

**12.** Dosage form according to any one of claims 1 to 11, containing

0.2 to 10 mg siponimod,
0.5 to 8 mg moisture-protective agent,
0 to 8 mg lubricant,

0 to 20 mg binder,
0 to 6 mg glidant,
0 to 15 mg disintegrant, and
17.8 to 84.3 mg filler.

**13.** Dosage form according to any one of claims 1 to 12, wherein the dosage form is a tablet having a content uniformity of 90 to 110%.

**14.** Dosage form according to any one of claims 1 to 13, wherein 0 to 4 wt.% of siponimod is decomposed after 6 months at 25°C at a humidity of 60%.

**15.** Process for producing a dosage form comprising the steps

i) providing siponimod,
ii) pre-blending the compound of step i) with moisture-protective agent and optionally pharmaceutical excipient,
iii) blending the mixture of step ii) with pharmaceutical excipient(s),
iv) processing the mixture of step iii) to a dosage form,
v) optionally, film-coating the dosage form.

**16.** Use of agglomerates comprising siponimod and moisture protective agent for producing a solid oral dosage form, having a shelf life at 25 °C of at least 2 years.

## Patentansprüche

**1.** Dosierungsform, die durch ein Verfahren erhältlich ist, das folgende Schritte umfasst:

i) Bereitstellen von Siponimod,
ii) Vormischung der Verbindung aus Schritt i) mit Feuchtigkeitsschutzmittel und gegebenenfalls pharmazeutischem Hilfsstoff,
iii) Mischen der Mischung aus Schritt ii) mit einem oder mehreren pharmazeutischem Hilfsstoffen,
iv) Verarbeiten der Mischung aus Schritt iii) zu einer Dosierungsform,
v) gegebenenfalls Filmbeschichten der Dosierungsform.

**2.** Dosierungsform nach Anspruch 1, enthaltend 0,2 bis 12 % (w/w) Siponimod, 1 bis 8 % (w/w) Feuchtigkeitsschutzmittel und 80 bis 98,8 % (w/w) eines oder mehrerer pharmazeutische Hilfsstoffe.

**3.** Dosierungsform nach Anspruch 1 oder 2, wobei Siponimod in einer Menge von 0,2 bis 10 mg, bezogen auf die Menge von Siponimod in Form der freien Base, vorliegt.

**4.** Dosierungsform nach einem der Ansprüche 1 bis 3, wobei die in-vitro-Freisetzung von Siponimod nach 30 Minuten nicht weniger als 80 % beträgt.

**5.** Dosierungsform nach einem der Ansprüche 1 bis 4, wobei das Verhältnis der Siponimod-Teilchengröße X90/X50 2 bis 5 beträgt.

**6.** Dosierungsform nach einem der Ansprüche 1 bis 5, wobei das Feuchtigkeitsschutzmittel einen n-Octanol/Wasser-Verteilungskoeffizienten (*log P)* von 0,1 bis 20, vorzugsweise 1 bis 15, aufweist.

**7.** Dosierungsform nach einem der Ansprüche 1 bis 6, wobei das Feuchtigkeitsschutzmittel aus hydriertem Pflanzenöl, Ricinusöl, Palmitolstearat, Glycerylpalmitostearat und Glycerylbehenat ausgewählt ist.

**8.** Dosierungsform nach Anspruch 7, wobei es sich bei dem Feuchtigkeitsschutzmittel um Glycerylbehenat handelt.

**9.** Dosierungsform nach einem der Ansprüche 1 bis 8, wobei die pharmazeutischen Hilfsstoffe aus Schmiermitteln, Bindemitteln, Gleitmitteln, Sprengmitteln und Füllstoffen ausgewählt sind.

**10.** Dosierungsform nach einem der Ansprüche 1 bis 9, wobei es sich bei dem Schmiermittel und dem Feuchtigkeitsschutzmittel um dieselbe Verbindung handelt, wobei die kombinierte Menge von Schmiermittel und Feuchtigkeitsschutzmittel 1 bis 16 % (w/w) beträgt.

**11.** Dosierungsform nach einem der Ansprüche 1 bis 10, enthaltend

0,2 bis 12 Gew.-% Siponimod,
1 bis 8 Gew.-% Feuchtigkeitsschutzmittel,
0 bis 8 Gew.-% Schmiermittel,
0 bis 10 Gew.-% Bindemittel,
0 bis 3 Gew.-% Gleitmittel,
0 bis 10 Gew.-% Sprengmittel und
49 bis 98,7 Gew.-% Füllstoff, bezogen auf das Gesamtgewicht der Dosierungsform.

**12.** Dosierungsform nach einem der Ansprüche 1 bis 11, enthaltend

0,2 bis 10 mg Siponimod,
0,5 bis 8 mg Feuchtigkeitsschutzmittel,
0 bis 8 mg Schmiermittel,
0 bis 20 mg Bindemittel,
0 bis 6 mg Gleitmittel,
0 bis 15 mg Sprengmittel und
17,8 bis 84,3 mg Füllstoff.

**13.** Dosierungsform nach einem der Ansprüche 1 bis 12, wobei es sich bei der Dosierungsform um eine Tablette mit einer Gehaltseinheitlichkeit von 90 bis 110 % handelt.

**14.** Dosierungsform nach einem der Ansprüche 1 bis 13, wobei nach sechs Monaten bei 25 °C und einer Feuchtigkeit von 60 % 0 bis 4 Gew.-% Siponimod zersetzt sind.

**15.** Verfahren zur Herstellung einer Dosierungsform, das folgende Schritte umfasst:

i) Bereitstellen von Siponimod,
ii) Vormischung der Verbindung aus Schritt i) mit Feuchtigkeitsschutzmittel und gegebenenfalls pharmazeutischem Hilfsstoff,
iii) Mischen der Mischung aus Schritt ii) mit einem oder mehreren pharmazeutischen Hilfsstoffen,
iv) Verarbeiten der Mischung aus Schritt iii) zu einer Dosierungsform,
v) gegebenenfalls Filmbeschichten der Dosierungsform.

**16.** Verwendung von Agglomeraten, die Siponimod und Feuchtigkeitsschutzmittel umfassen, zur Herstellung einer festen oralen Dosierungsform mit einer Haltbarkeit von mindestens 2 Jahren bei 25 °C.

## Revendications

**1.** Forme pharmaceutique pouvant être obtenue par un procédé comprenant les étapes de

i) fourniture de siponimod,
ii) prémélange du composé de l'étape i) avec un agent de protection contre l'humidité et facultativement un excipient pharmaceutique,
iii) mélange du mélange de l'étape ii) avec un ou plusieurs excipient(s) pharmaceutique(s),
iv) traitement du mélange de l'étape iii) pour obtenir une forme pharmaceutique,
v) facultativement, pelliculage de la forme pharmaceutique.

**2.** Forme pharmaceutique selon la revendication 1 contenant 0,2 à 12 % (p/p) de siponimod, 1 à 8 % (p/p) d'agent de protection contre l'humidité et 80 à 98,8 % (p/p) d'excipient(s) pharmaceutique(s).

**3.** Forme pharmaceutique selon la revendication 1 ou 2, dans laquelle le siponimod est présent en une quantité de 0,2 à 10 mg, sur la base de la quantité de siponimod sous forme de base libre.

4. Forme pharmaceutique selon l'une quelconque des revendications 1 à 3, dans laquelle la libération *in vitro* de siponimod n'est pas inférieure à 80 % après 30 minutes.

5. Forme pharmaceutique selon l'une quelconque des revendications 1 à 4, dans laquelle le rapport de la taille de particule de siponimod X90/X50 est de 2 à 5.

6. Forme pharmaceutique selon l'une quelconque des revendications 1 à 5, dans laquelle l'agent de protection contre l'humidité présente un coefficient de partage n-octanol/eau (*log P*) de 0,1 à 20, de préférence 1 à 15.

7. Forme pharmaceutique selon l'une quelconque des revendications 1 à 6, dans laquelle l'agent de protection contre l'humidité est choisi parmi une huile végétale hydrogénée, l'huile de ricin, le stéarate de palmitol, le palmitostéarate de glycéryle et le béhénate de glycéryle.

8. Forme pharmaceutique selon la revendication 7, dans laquelle l'agent de protection contre l'humidité est le béhénate de glycéryle.

9. Forme pharmaceutique selon l'une quelconque des revendications 1 à 8, dans laquelle les excipients pharmaceutiques sont choisis parmi des lubrifiants, des liants, des agents glissants, des délitants et des charges.

10. Forme pharmaceutique selon l'une quelconque des revendications 1 à 9, dans laquelle le lubrifiant et l'agent de protection contre l'humidité sont le même composé, dans laquelle la quantité combinée de lubrifiant et d'agent de protection contre l'humidité est de 1 à 16 % (p/p).

11. Forme pharmaceutique selon l'une quelconque des revendications 1 à 10, contenant

0,2 à 12 % en poids de siponimod,
1 à 8 % en poids d'agent de protection contre l'humidité,
0 à 8 % en poids de lubrifiant,
0 à 10 % en poids de liant,
0 à 3 % en poids d'agent glissant,
0 à 10 % en poids de délitant, et
49 à 98,7 % en poids de charge, sur la base du poids total de la forme pharmaceutique.

12. Forme pharmaceutique selon l'une quelconque des revendications 1 à 11, contenant

0,2 à 10 mg de siponimod,
0,5 à 8 mg d'agent de protection contre l'humidité,
0 à 8 mg de lubrifiant,
0 à 20 mg de liant,
0 à 6 mg d'agent glissant,
0 à 15 mg de délitant, et
17,8 à 84,3 mg de charge.

13. Forme pharmaceutique selon l'une quelconque des revendications 1 à 12, la forme pharmaceutique étant un comprimé ayant une uniformité de teneur de 90 à 110 %.

14. Forme pharmaceutique selon l'une quelconque des revendications 1 à 13, dans laquelle 0 à 4 % en poids de siponimod est décomposé après 6 mois à 25 °C à une humidité de 60 %.

15. Procédé de production d'une forme pharmaceutique comprenant les étapes de

i) fourniture de siponimod,
ii) prémélange de la composé de l'étape i) avec un agent de protection contre l'humidité et facultativement un excipient pharmaceutique,
iii) mélange du mélange de l'étape ii) avec un ou plusieurs excipient(s) pharmaceutique(s),
iv) traitement du mélange de l'étape iii) pour obtenir une forme pharmaceutique,
v) facultativement, pelliculage de la forme pharmaceutique.

16. Utilisation d'agglomérats comprenant du siponimod et un agent de protection contre l'humidité pour produire une forme pharmaceutique orale solide, ayant une durée de conservation à 25 °C d'au moins 2 ans.

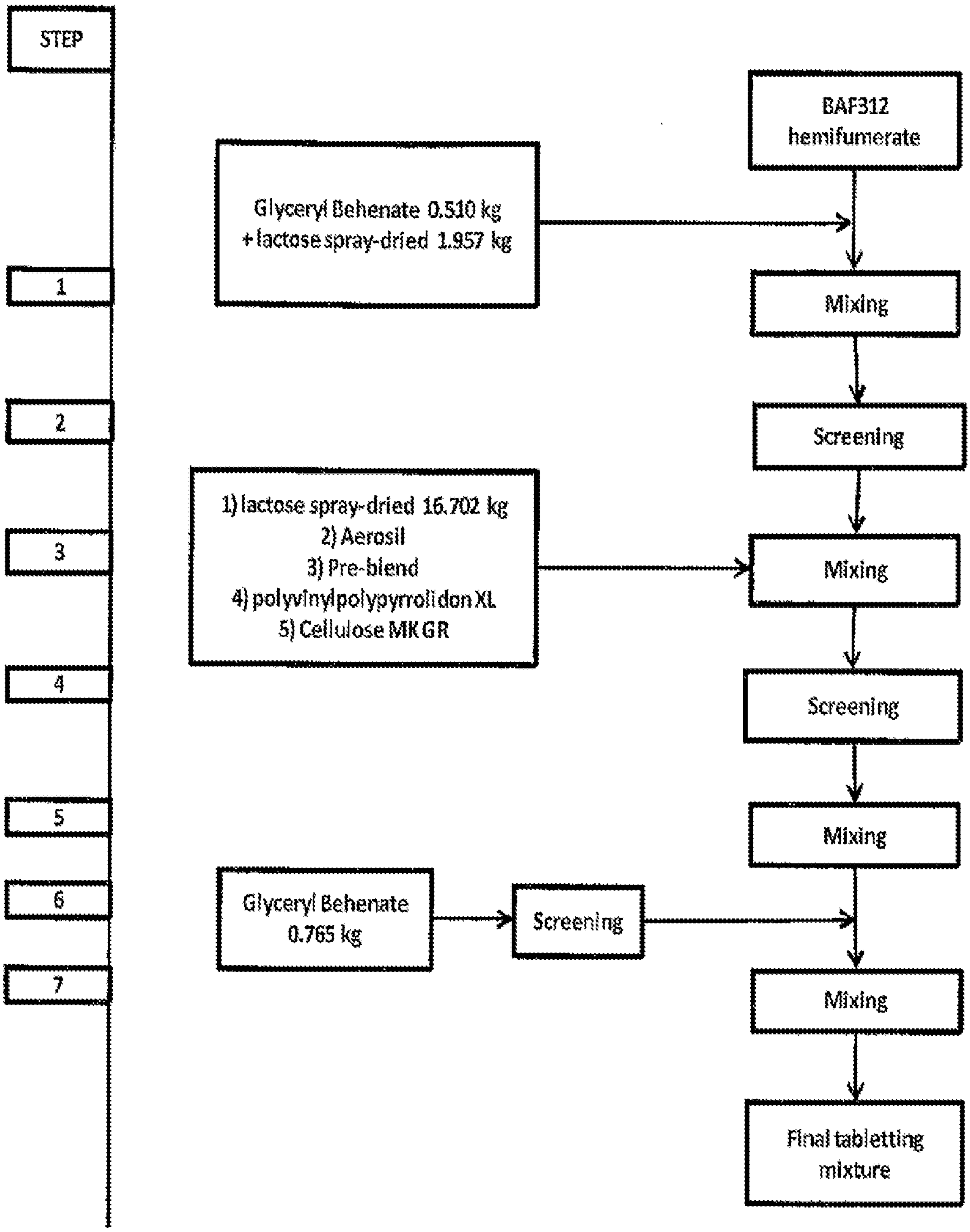
STEP
1
2
3
4
5
6
7
BAF312 hemifumerate
Glyceryl Behenate 0.510 kg + lactose spray-dried 1.957 kg
Mixing
Screening
1) lactose spray-dried 16.702 kg
2) Aerosil
3) Pre-blend
4) polyvinylpolypyrrolidon XL
5) Cellulose MK GR
Mixing
Screening
Mixing
Glyceryl Behenate 0.765 kg
Screening
Mixing
Final tabletting mixture

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012093161 A1
- WO 2010071794 A
- WO 201008045 A
- WO 2010080409 A


### Non-patent literature cited in the description


- Ph. Eur.